# EUROPÄISCHE PATENTANMELDUNG

(11) **EP 2 826 850 A1**
(43) Veröffentlichungstag der Anmeldung: **21.01.2015**
(21) Anmeldenummer: 14177281.4
(22) Anmeldetag: 16.07.2014
(51) Int. Cl.: C12M 1/107, C12M 1/34, C12M 1/00

(54) **Biogasanlage zur Erzeugung von Biogas aus nicht-pumpbarer Biomasse sowie Verfahren zu ihrem Betrieb**

(30) Priorität: 18.07.2013 DE 102013107683
(71) Anmelder: Lutz, Peter, 81925 Unterföhring (DE)
(72) Erfinder: Lutz, Peter, 81925 Unterföhring (DE)
(74) Vertreter: Alber, Norbert

(57) **Zusammenfassung**

Das in bestimmten Betriebszuständen notwendige Spülen von Fermentern mit einem unbrennbaren Spülgas erfolgt erfindungsgemäß nicht mit dem Abgas aus dem biogasbetriebenen Gasmotor, sondern mit dem Abgas einer zusätzlichen Verbrennungsvorrichtung, in der Regel einem einfachen Heizkessel, in dem ein Brennstoff mit definierter Zusammensetzung, beispielsweise Erdgas, verbrannt wird. Durch diese zusätzliche Verbrennungsvorrichtung kann die gewünschte Menge an Spülgas unabhängig vom Anfall an Biogas passend erzeugt werden und das Abgas besitzt eine bekannte Zusammensetzung ohne unerwünschte Schadstoffe. Zusätzlich dient der Heizkessel als teil-redundante Verwertungsmöglichkeit für das anfallende Biogas, falls der Gasmotor nicht einsatzfähig ist.

## Beschreibung

### I. Anwendungsgebiet

Die Erfindung betrifft eine Biogasanlage zur Erzeugung von Biogas aus nicht-pumpbarer Biomasse sowie ein Verfahren zu ihrem Betrieb.

### II. Technischer Hintergrund

Heute wird Biomasse, beispielsweise Silage oder Bioabfälle oder die organische Fraktion des Hausmülls, auf zwei grundsätzlich unterschiedliche Arten anaerob vergoren:
- einerseits im Nassgär-Verfahren, bei dem die Biomasse in Form einer pumpfähigen Schlempe vorliegt,
- andererseits im hier vorliegenden Feststoff-Verfahren, bei der die Biomasse, nur durch das Animpfen mit bereits vergorenem Material, in die Fermenter eingebracht wird.

Dabei sammelt sich das entstehende, brennbare Biogas im Freiraum oberhalb der Biomasse in jedem Fermenter und kann von dort aus abgepumpt und einem Verbraucher zugeführt werden.

In diesem Zusammenhang ist es sehr wichtig, dass in dem Fermenter keine Luft vorhanden ist oder von außen eindringen kann, denn das sich bildende Biogas könnte dann zusammen mit dem Luftsauerstoff ein explosives Gasgemisch bilden.

Zum einen ist deshalb die luft- und gasdichte Verschließbarkeit des Fermenters mittels einer entsprechenden Schließvorrichtung wichtig, um das Eindringen von Luft von außen zu verhindern. Aus dem gleichen Grund wird im Inneren des Fermenters auch ständig ein leichter Überdruck von **5** mbar bis **30** mbar aufrecht erhalten, um auch bei kleinen auftretenden Undichtigkeiten ein Eindringen von Luft ins Innere des Fermenters zu verhindern.

Zusätzlich wird die Gaszusammensetzung im Fermenter ständig kontrolliert, beispielsweise hinsichtlich des Sauerstoffanteils oder des Kohlendioxidanteils und/oder des Methananteils, um bei entstehendem explosiven Gasgemisch Gegenmaßnahmen ergreifen zu können.

Weitere kritische Situationen sind der Beginn und das Ende des Umsetzungsprozesses im Fermenter:
Nach dem Befüllen des Fermenters mit Biomasse befindet sich im Freiraum oberhalb der Biomasse Luft im Fermenter, die nach Schließen der Schließvorrichtung erst vollständig aus dem Fermenter entfernt werden muss, um eine Vermischung mit dem sich bildenden Biogas zu vermeiden.

Ebenso würde beim Öffnen des Fermenters nach Ende des Umsetzungsprozesses - um die Gärreste zu entnehmen - Luft in den Fermenter einströmen, und falls dann noch Biogas im Fermenter vorhanden ist, sich mit dem Biogas zu einem explosionsfähigen Gemisch verbinden können.

Deshalb muss in beiden Fällen, also nach dem Befüllen und Verschließen des Fermenters und vor dem Öffnen des Behälters, der Gasraum im Fermenter oberhalb der Biomasse mit einem unbrennbaren Spülgas gespült werden, um explosionsfähige Komponenten aus dem Freiraum hinaus zu pressen. Hierfür wird in aller Regel das Abgas des Biogas-Verbrauchers, in der Regel eines Gasmotors, der einen Generator zur Stromerzeugung antreibt, benutzt.

Diese Lösung weist jedoch mehrere Nachteile auf:
Zum einen hängt die Zusammensetzung des Abgases, insbesondere die enthaltenen Schadstoffe, aus dem Biogasverbraucher davon ab, aus welcher Art von Biomasse das im Biogasverbraucher verbrannte Biogas hergestellt wurde, also beispielsweise ob es sich um nachwachsende Rohstoffe oder den Bioanteil aus z.B. städtischem Müll handelt. Da selbst in ein und derselben Biogasanlage die unterschiedlichen Fermenter mit unterschiedlicher Art von Biomasse gefüllt sein können, und die Gärprozesse der einzelnen Fermenter zeitversetzt zueinander ablaufen, kann sich die Zusammensetzung des gesammelten Biogases, welches dem Biogasverbraucher zugeführt wird, zeitabhängig relativ stark ändern.

Die im Abgas des Biogasverbrauchers enthaltenen Schadstoffe werden auf diese Art und Weise in den oder die gespülten Fermenter eingebracht. Dies ist aus arbeitsschutzrechtlichen Gründen für die danach im Fermenter arbeitenden Personen kritisch, und dies kann auch die im Fermenter vorhandene Biomasse bzw. Gärreste negativ beeinflussen.

Ferner wird das während der Spülung mit Abgas aus dem Fermenter abgesaugte Gasgemisch - abhängig von der Zusammensetzung, insbesondere dem Methan-Gehalt - häufig über einen Spülgas-Abluftkamin in die Umgebung entlassen.

Da das zum Spülen verwendete Abgas des Biogasverbrauchers vorher gekühlt wird bis herunter auf **100**° C oder sogar weniger, besitzt das aus dem Spülgas-Abluftkamin aufsteigende Gasgemisch nur einen geringen Auftrieb, und kühlt sich in der Umgebungsluft sehr schnell weiter ab mit der Folge, dass es bereits in der näheren Umgebung des Abluftkamins zu Boden sinkt, also noch in dem Bereich, in dem das Personal der Biogasanlage tätig ist, und vor allem wegen der geringen Vermischung mit Umgebungsluft noch in relativ konzentrierter Form. Auch dies ist unter Emissionsgesichtspunkten kritisch.

Der andere Nachteil besteht darin, dass ein Gasmotor, wie er üblicherweise als Biogasverbraucher benutzt wird, mit sehr konstanter Drehzahl laufen muss, beispielsweise **1500** Hz, damit der angeschlossene Generator Wechselstrom erzeugt mit einer Frequenz, die problemlos in das Stromnetz eingespeist werden kann.

Zur Regelung des Gasmotors steht einerseits eine Drosselklappe zur Verfügung, die die zugeführte Menge an Gas regelt, und ein Gemischregler, der dem Biogas die passende Menge an Luft zumischt, abhängig von der Qualität, also in der Regel dem Methangehalt, des Biogases. Hierüber wird also bei Bedarf nicht die Drehzahl des Gasmotors verändert, sondern die von ihm abgegebene Leistung.

Dies bewirkt auf der Zuführungsseite, dass ein solcher Gasmotor schnelle Veränderungen, beispielsweise des Methangehaltes des Biogases, unter Umständen nicht nachregeln kann, was Schwierigkeiten auf der Seite der Netzeinspeisung nach sich zieht. Dies bewirkt auf der Abgasseite aber auch, dass im Normalbetrieb der Gasmotor nicht immer die gleiche Menge an Abgas abgibt, sondern abhängig von der Leistung, mit der der Gasmotor läuft, auch die erzeugte Menge an Abgas.

Der Spülgasbedarf einer Biogasanlage dagegen ist dann, wenn ein Fermenter gespült werden soll, sehr hoch, denn die Spülung soll ja mit möglichst großem Spülgas-Durchsatz und damit in möglichst kurzer Zeit abgeschlossen werden. Falls es dazwischen Zeiten gibt, in denen kein Fermenter gespült werden muss, wird auch manchmal überhaupt kein Spülgas benötigt

Der Abgas-Anfall des Gasmotors und der Spülgasbedarf der Biogasanlage stimmen also von vornherein selten automatisch überein.

Dabei muss auch berücksichtigt werden, dass nicht das ganze Abgasvolumen als Spülgas zur Verfügung steht, sondern aus dem Abgaskamin des Gasmotors nur ein Teil des Abgases abgezogen werden kann, um zuverlässig zu verhindern, dass beim Abfördern des Abgases als Spülgas nicht unbeabsichtigt Luft über den Abgaskamin angesaugt und dem Spülgas beigefügt wird, was ja zur Vermeidung explosiver Gasgemische gerade vermieden werden soll.

Das vollständige Verschließen des Abgaskamins ist ebenfalls nicht möglich, da die Druckverhältnisse im Verbrennungstrakt und Abgastrakt des Gasmotors hierdurch unzulässig verändert würden.

Ein weiterer Nachteil besteht darin, dass bei Vorhandensein eines einzigen Gasmotors keine Wärmeversorgung für die Fermenter zur Verfügung steht, falls dieser einzige Gasmotor ausfällt. Die Alternative ist eine redundante Bestückung mit zwei Gasmotoren, was jedoch die Investitions- und Unterhaltskosten der Biogasanlage in die Höhe treibt.

### III. Darstellung der Erfindung

### a) Technische Aufgabe

Es ist daher die Aufgabe gemäß der Erfindung, eine Biogasanlage und ein Verfahren zu ihrem Betrieb zur Verfügung zu stellen, bei dem trotz geringer Investitionskosten und hoher Ausfallsicherheit der Anlage eine zuverlässige Spülung der Fermenter mit unbrennbarem Spülgas gewährleistet ist und auch die Wärmeversorgung der Fermenter sichergestellt ist.

### b) Lösung der Aufgabe

Diese Aufgabe wird durch die Merkmale der Ansprüche **1** und **6** gelöst. Vorteilhafte Ausführungsformen ergeben sich aus den Unteransprüchen.

Hinsichtlich des **Verfahrens** zum Betrieb der Biogasanlage wird diese Aufgabe dadurch gelöst, dass zum Spülen eines oder mehrerer Fermenter mit einem nicht brennbaren Spülgas nicht das Abgas des regulären. insbesondere des momentan Biogas verbrennenden Biogasverbrauchers, in der Regel eines Gasmotors, verwendet wird, sondern das Abgas aus der Verbrennung von einer Verbrennungsvorrichtung, die (insbesondere) zusätzlich zu dem wenigstens einen, insbesondere momentan Biogas verbrauchenden, Biogas-Verbraucher vorhanden ist, und in der nicht das erzeugte Biogas, sondern ein anderer Brennstoff definierter und damit auch jederzeit bekannter gleichbleibender Zusammensetzung, insbesondere ein fossiler Brennstoff wie etwa Erdgas oder Flüssiggas, verbrannt wird, um das daraus entstehende Abgas als Spülgas zu benutzen.

Auf diese Art und Weise ist wegen der bekannten Zusammensetzung des Brennstoffes auch die Zusammensetzung des Abgases bekannt, so dass das unzulässige Einleiten von Schadstoffen über das Spülgas mittels des Spülgases in die Fermenter vermieden wird.

Die zusätzliche Verbrennungsvorrichtung ist vorzugsweise ein Heizkessel, in dem durch die Verbrennung nicht nur das als Spülgas benötigte Abgas erzeugt wird, sondern durch Aufheizen eines Wärmeträgermediums, in der Regel ein Gemisch auf Wasserbasis, auch Wärme erzeugt wird, wie sie beispielsweise zum Beheizen der Fermenter zumindest in der Anfangsphase des Gärprozesses benötigt wird.

Die Menge des in dieser Verbrennungsvorrichtung verbrannten Brennstoffes bekannter Zusammensetzung wird gesteuert in Abhängigkeit der benötigten Menge an Spülgas und/oder an Wärme.

Vorzugsweise wird an den Biogas-Entnahmeöffnungen der einzelnen Fermenter und/oder an der Biogas-Sammelleitung vor der Einleitung in den Biogas-Verbraucher ständig die Qualität, insbesondere der Methangehalt oder die sonstige Zusammensetzung, des Biogases gemessen, um abhängig davon die Einleitung der Spülgasmenge, insbesondere pro Zeiteinheit, in die einzelnen Fermenter zu steuern.

Dabei wird der Zeitversatz berücksichtigt, den das Spülgas für die Durchströmung eines Fermenters benötigt aufgrund der Tatsache, dass Spülgas-Zufuhröffnung und Biogas-Entnahmeöffnung in der Regel sich in den Fermentern diagonal gegenüber liegen, also soweit wie möglich voneinander beabstandet sind, damit auf dem Weg dazwischen eine möglichst lange Zeitspanne für das Mischen von Spülgas und Biogas zur Verfügung steht.

Weiterhin kann in der zusätzlichen Verbrennungsvorrichtung, insbesondere dem Heizkessel, auch das in der Biogasanlage entstehende Biogas verbrannt werden und zu Wärme und Abgas umgewandelt werden, falls der reguläre Biogas-Verbraucher, in der Regel ein Gasmotor, wegen Wartungsarbeiten oder Defekt nicht einsatzbereit ist. Zwar kann dann kein Strom erzeugt werden, aber es kann zumindest die Beheizung der Fermenter und/oder eine unbedingt benötigte Spülung der Fermenter - wenn auch mit Abgas nicht ganz bekannter Zusammensetzung - weiterhin zur Verfügung gestellt werden. Möglicherweise wird dafür weniger Biogas benötigt, als anfällt, aber die Differenz kann gegebenenfalls über eine ausreichend lange Zeitspanne zwischengepuffert werden, bis der reguläre Biogasverbraucher zum Zwecke der Stromerzeugung wieder einsatzfähig ist.

Hinsichtlich der **Biogasanlage** wird die erfindungsgemäße Aufgabe dadurch gelöst, dass eine Verbrennungsvorrichtung vorhanden ist, die umschaltbar ist von der Verbrennung auf Biogas auf andere Brennstoffe, insbesondere Brennstoffe mit bekannter Zusammensetzung, beispielsweise fossile Brennstoffe wie Erdgas oder Flüssiggas. Diese Verbrennungsvorrichtung ist insbesondere zusätzlich zum regulären Biogas-Verbraucher, welches in der Regel ein Gasmotor ist, vorhanden.

Wenn die zusätzliche Verbrennungsvorrichtung zusätzlich zum regulären Biogas-Verbraucher vorhanden ist, erreicht die Biogasanlage dadurch zwar keine Redundanz für die Stromerzeugung, aber eine **100**%ige Redundanz für die Wärme-und Abgaserzeugung:
Eine **50**%ige Redundanz für die Stromerzeugung ist dann gegeben, wenn als Biogas-Verbraucher zwei Maschinen, also zum Beispiel zwei Gasmotoren, nebeneinander betrieben werden, um bei Ausfall einer der beiden Maschinen mit der anderen weiterhin Biogas verwerten zu können, und beispielsweise Strom über den angeschlossenen Generator erzeugen zu können.
Der Nachteil einer solchen **50**%igen Redundanz für die Stromerzeugung besteht darin, dass die Effizienz der Anlage sinkt, da
   - die Betriebs- und Wartungskosten für zwei kleine Maschinen für je 50%ige Biogas-Verwertungsleistung größer sind als für eine große Maschine,
   - der Wirkungsgrad einer großen Maschine hinsichtlich Abwärmeverluste, Reibungsverluste etc. besser ist als in der Summe zweier kleiner Maschinen und
   - die Investitionskosten zweier kleiner Maschinen wesentlich höher sind als die einer großen Maschine, auch unter dem Gesichtspunkt des Platzbedarfs.

Umfasst die Biogasanlage dagegen nur einen regulären Biogas-Verbraucher, beispielsweise einen Gasmotor, und zusätzlich statt eines zweiten Gasmotors nur eine einfache zusätzliche Verbrennungsvorrichtung wie etwa einen Heizkessel, so sind die Investitionskosten deutlich geringer, da die Kosten eines solchen Heizkessels weit unter der eines Gasmotors liegen.

Dennoch wird eine teilweise Redundanz erreicht, denn bei Ausfall des regulären Biogas-Verbrauchers kann das anfallende Biogas auch in der zusätzlich vorhandenen Verbrennungsvorrichtung, dem Heizkessel, verbrannt werden. Dort wird zwar damit kein Strom erzeugt, der verkauft werden kann, sondern lediglich Wärme und Abgas, so dass aus diesem Grund in dieser zusätzlichen Brennvorrichtung auch nur so viel Biogas verbrannt werden wird, um den vorhandenen Bedarf an Wärme für die Beheizung der Fermenter oder einen zu versorgenden Wärmeverbraucher zu decken und/oder um den Bedarf als Spülgas in Form von Abgas zu decken.

In der Regel wird jedoch mehr Biogas anfallen, es ist jedoch leichter möglich, lediglich die Differenz zwischen dem verbrannten Biogas und dem anfallenden Biogas über eine begrenzte Zeit zu puffern oder abzufackeln, bis der Gasmotor wieder einsatzfähig ist, als die gesamte anfallende Menge an Biogas.

Theoretisch könnte also zur Erzeugung von Spülgas definierter Zusammensetzung auch der reguläre Biogas-Verbraucher, in der Regel ein Gasmotor, sofern er umschaltbar ist, zeitweise statt mit Biogas mit dem Brennstoff definierter Zusammensetzung betrieben werden, um das entstehende Abgas als Spülgas definierter Zusammensetzung für die Fermenter zu benutzen. Eine Redundanz der Anlage ist dann jedoch nicht mehr gegeben, denn bei Ausfall dieses regulären Biogas-Verbrauchers als einziger Verbrennungsvorrichtung ohne eine zusätzliche weitere Verbrennungsvorrichtung kann weder Spülgas noch Wärme erzeugt werden.

Eine andere Möglichkeit besteht darin, dass die Biogasanlage einen Anschluss für einen mobilen Biogas-Verbraucher, in der Regel einen Biogas-Motor mit angeschlossenem elektrischen Generator, besitzt. Dann kann bei Ausfall des fest installierten Biogas-Verbrauchers ein solcher mobiler Biogas-Verbraucher herangeschafft und angeschlossen werden und kann das anfallende Biogas weiterhin verwerten.

Wenn ein solcher Anschluss auch zum Anschließen einer mobilen zusätzlichen Verbrennungsvorrichtung geeignet ist, muss die erfindungsgemäße vorhandene zusätzliche Verbrennungsvorrichtung nicht stationär an einer Biogasanlage vorhanden sein, sondern kann für mehrere Biogasanlagen im Verbund genutzt werden.

### c) Ausführungsbeispiele

Ausführungsformen gemäß der Erfindung sind im Folgenden beispielhaft näher beschrieben. Es zeigt:

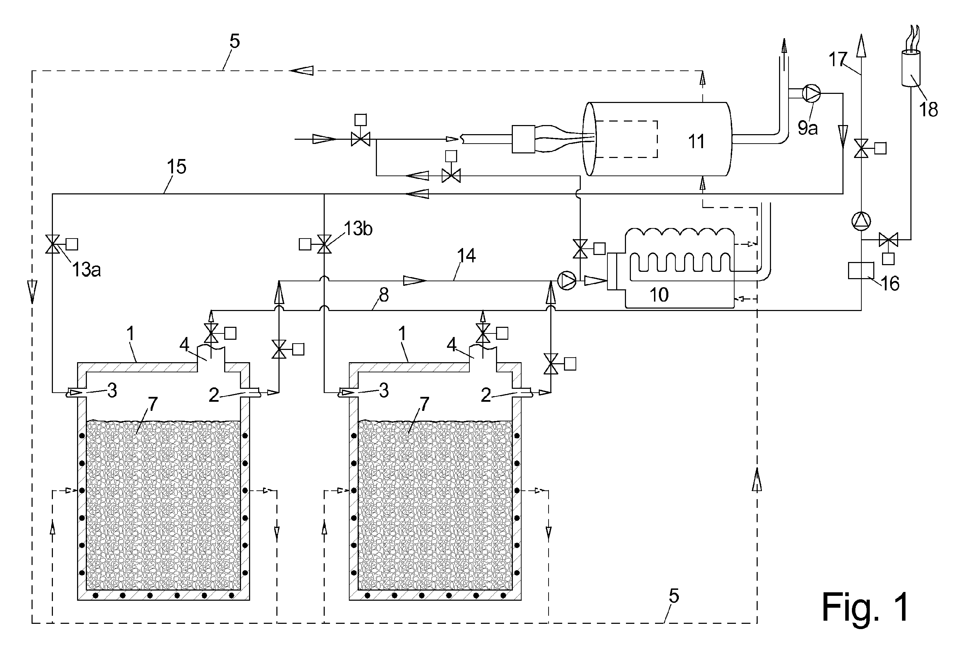

- Fig. **1:**: eine Prinzipdarstellung der Biogasanlage.

Dabei sind die Fermenter **1,** von denen hier nur zwei dargestellt sind, im Schnitt bzw. mit offener Frontseite dargestellt, sodass sichtbar ist, dass die beiden dargestellten Fermenter **1** fast vollständig mit Biomasse **7** gefüllt sind.

In den Wänden und im Boden der Fermenter **1** sind Heizungsrohre **6** eingegossen, die für jeden Fermenter **1** separat über Stichleitungen mit dem Wärmekreislauf **5** verbunden sind.

Dargestellt ist ferner der Gasmotor **10,** der als regulärer Biogas-Verbraucher dient, sowie ein zusätzlicher Heizkessel **11,** der mittels einer Flamme beheizt werden kann, die wahlweise mittels einem definierten von z. B. außerhalb der Biogasanlage angelieferten Brennstoff **12,** wie etwa Flüssiggas, betrieben werden kann oder auch mittels Biogas aus der Biogas-Sammelleitung **14,** die mit den Biogas-Entnahmeöffnungen **2** jedes Fermenters **1** in Verbindung steht und von dort das entstehende Biogas absaugt und normalerweise dem Gasmotor **10** zuführt. Über eine Abzweigung kann dieses Biogas auch dem Heizkessel **11** zugeführt werden.

Der Heizkessel wird über eine Versorgungsleitung mit dem Brennstoff, der kein Biogas ist, versorgt.

Das erfindungsgemäß Wesentliche besteht darin, dass der Abgasleitung des Heizkessels **11** über einen Abzweig Abgas entnommen und über eine Spülgas-Versorgungsleitung **15** den Spülgas-Zufuhröffnungen **3** jedes Fermenters zugeführt werden kann.

Falls auf diese Art und Weise der Gasraum eines der Fermenter **1** oberhalb der Biomasse **7** mit Abgas aus dem Heizkessel **11** gespült werden soll, wird das entsprechende Sperrventil, z.B. **13**a, geöffnet und das Gebläse **9** im Abzweig der Abgasleitung des Heizkessels **11** in Gang gesetzt. Das sich im Gasraum des Fermenters **1** bildende Gemisch aus Abgas und Biogas wird über die Spülgas-Entnahmeöffnung **4** abgesaugt und - je nach Zusammensetzung dieses Schwachgasgemisches, die durch eine Analyseeinheit **16** ermittelt wird - über eine Schwachgas-Sammelleitung **8**entweder dem Spülgas-Abluftkamin **17** oder Fackel **18** zum Verbrennen dieses Gasgemisches zugeführt.

Der Wärmekreislauf **5** steht sowohl mit dem Heizkessel **11** als auch mit dem Gasmotor **10** in Verbindung und nimmt von dort jeweils Wärme auf.

Der Heizkessel **11** wird also immer dann in Gang gesetzt und mit einem Brennstoff **12** definierter Zusammensetzung betrieben, wenn zum Spülen eines der Fermenters **1** Spülgas definierter Zusammensetzung benötigt wird.

Der Heizkessel **11** wird auch dann in Gang gesetzt und mit dem Brennstoff **12** definierter Zusammensetzung betrieben, wenn beim Anfahren der Biogasanlage die Fermenter über den Wärmekreislauf **5** beheizt werden müssen, aber mangels ausreichendem Anfall von Biogas noch keine ausreichende Abwärme vom Gasmotor **10** hierfür zur Verfügung steht.

Der Heizkessel **11** kann auch dann in Betrieb gesetzt werden und mit Biogas aus der Biogas-Sammelleitung **14** betrieben werden, falls der Gasmotor **10** ausgefallen ist, und dennoch Wärme und/oder Abgas zum Beheizen und/oder Spülen der Fermenter **1** benötigt wird.

### BEZUGSZEICHENLISTE

- **1**: Fermenter
- **2**: Biogas-Entnahmeöffnung
- **3**: Spülgas-Zufuhröffnung
- **4**: Spülgas-Entnahmeöffnung
- **5**: Wärmekreislauf
- **6**: Heizrohr
- **7**: Biomasse
- **8**: Schwachgas-Sammelleitung
- **9**: Gebläse
- **10**: Gasmotor, Biogas-Verbraucher
- **11**: Heizkessel, Verbrennungsvorrichtung
- **12**: definierter Brennstoff
- **13**a, b: Sperrventil
- **14**: Biogas-Sammelleitung
- **15**: Spülgas-Versorgungsleitung
- **16**: Analyseeinheit
- **17**: Spülgas-Abluftkamin
- **18**: Fackel

## Patentansprüche

1. Verfahren zur Erzeugung von Biogas aus nicht-pumpbarer Biomasse **(7),** mit ist
- mehreren gas- und flüssigkeitsdicht verschließbaren Fermentern **(1)** für die Biomasse **(7),**
- einer Biogas-Entnahmeöffnung **(2)** in jedem Fermenter **(1),**
- einer Spülgas-Zufuhröffnung **(3)** in jedem Fermenter **(1),**
- wenigstens einem Biogas-Verbraucher **(10),**
**dadurch gekennzeichnet, dass**
das Spülen eines Fermenters **(1)** mit einem unbrennbaren Gas mittels der Abgase aus einer insbesondere gegenüber den oben genannten Komponenten zusätzlichen Verbrennungsvorrichtung **(11)** durchgeführt wird, wobei diese mit Brennstoffen **(12)** definierter Zusammensetzung betrieben wird.

2. Verfahren nach Anspruch **1,**
**dadurch gekennzeichnet, dass**
die Brennstoffe **(12)** definierter Zusammensetzung fossile Brennstoffe, insbesondere Erdgas oder Flüssiggas, sind.

3. Verfahren nach einem der vorhergehenden Ansprüche,
**dadurch gekennzeichnet, dass**
die zusätzliche Verbrennungsvorrichtung **(11)** außer Abgas auch Wärme, insbesondere in Form eines aufgeheizten Wärmeträgermediums, erzeugt, und das Wärmeträgermedium insbesondere den Fermentern **(1)** zum Beheizen zugeführt wird.

4. Verfahren nach einem der vorhergehenden Ansprüche,
**dadurch gekennzeichnet, dass**
im Falle des Ausfalls des Biogasverbrauchers **(10)** das anfallende Biogas der zusätzlichen Verbrennungsvorrichtung **(11)** zugeführt wird.

5. Verfahren nach einem der vorhergehenden Ansprüche,
**dadurch gekennzeichnet, dass**
vom Abgas der Verbrennungsvorrichtung **(11)** ein Teil als Spülgas für die Fermenter (1) so abgezweigt wird, dass
- der als Spülgas verwendete Anteil maximal **90** %, besser maximal **80** % des anfallenden Abgases der Verbrennungsvorrichtung **(11)** ist und/oder
- der Druck in der Spülgas-Versorgungsleitung **(15)** höher ist als der Druck in den damit zu beschickenden Fermentern **(1).**

6. Biogasanlage zur Erzeugung von Biogas aus nicht-pumpbarer Biomasse, mit
- mehreren gas- und flüssigkeitsdicht verschließbaren Fermentern **(1)** für die Biomasse **(7),**
- einer Biogas-Entnahmeöffnung **(2)** in jedem Fermenter **(1),**
- einer Spülgas-Zufuhröffnung **(3)** in jedem Fermenter **(1),**
- einem Biogas-Verbraucher **(10),**
**gekennzeichnet durch**
- eine insbesondere zusätzliche Verbrennungsvorrichtung **(11),** die sowohl Biogas als auch andere Brennstoffe **(12)** mit insbesondere definierter Zusammensetzung verbrennen kann und
- die mit ihrer Abgasleitung mit den einzelnen Fermentern **(1)** verbunden ist.

7. Biogasanlage nach Anspruch **6,**
**dadurch gekennzeichnet, dass**
die Verbrennungsvorrichtung **(11)** ein Heizkessel **(11)** ist.

8. Biogasanlage nach einem der vorhergehenden Vorrichtungsansprüche,
**dadurch gekennzeichnet, dass**
in der Spülgas-Versorgungsleitung **(15)** von der Abgasleitung der Verbrennungsvorrichtung zu den Fermentern **(1)** ein steuerbares Gebläse **(9)** angeordnet ist, das mit einer Steuerung gekoppelt ist, die in der Lage ist, so zu steuern, dass
- der als Spülgas verwendete Anteil maximal **90** %, besser maximal **80** % des anfallenden Abgases der Verbrennungsvorrichtung **(11)** ist und/oder
- der Druck in der Spülgas-Versorgungsleitung **(15)** höher ist als der Druck in den damit zu beschickenden Fermentern **(1).**

9. Biogasanlage nach einem der vorhergehenden Vorrichtungsansprüche,
**dadurch gekennzeichnet, dass**
die Verbrennungsvorrichtung **(11)** mit den Biogas-Entnahmeöffnungen **(2)** der Fermenter **(1)** verbindbar ist.

10. Biogasanlage nach einem der vorhergehenden Vorrichtungsansprüche,
**dadurch gekennzeichnet, dass**
die Biogasanlage einen Anschluss für einen mobilen Biogas-Verbraucher, insbesondere einen Gasmotor mit angekoppeltem elektrischen Generator, aufweist.
